Europäisches Patentamt

European Patent Office

Office européen des brevets

(11) Publication number: **0 182 240**
**B1**

(12) **EUROPEAN PATENT SPECIFICATION**

(45) Date of publication of patent specification: 25.01.89

(51) Int. Cl.⁴: **A 61 K 39/09**

(21) Application number: **85114311.5**

(22) Date of filing: **11.11.85**

(54) **Preparation of enzyme-detergent extracted streptococcus equi vaccine.**

(30) Priority: **23.11.84 US 674449**

(43) Date of publication of application:
**28.05.86 Bulletin 86/22**

(45) Publication of the grant of the patent:
**25.01.89 Bulletin 89/04**

(84) Designated Contracting States:
**BE CH DE FR GB IT LI NL SE**

(56) References cited:
**US-A-3 852 420**

**CHEMICAL ABSTRACTS, vol. 90, no. 23, 4th June 1979, Columbus, Ohio (US); K.NAMBA: "Isolation of M protein from the cell envelope of group A streptococcus pyogenes by use of a cell wall lytic enzyme", p. 310, no. 182888j**

**CHEMICAL ABSTRACTS, vol. 98, no. 14, 4th April 1983, Columbus, Ohio (US); p. 387, no. 113700v**

**CHEMICAL ABSTRACTS, vol. 85, no. 13, 27th September 1976, Columbus, Ohio (US); V.A.FISCHETTI et al.: "Streptococcal M protein extracted by nonionic detergent. I. Properties of the antiphagocytic and type-specific molecules", p. 452, no. 92010j**

(73) Proprietor: **MILES INC.**
**1127 Myrtle Street**
**Elkhart Indiana 46514 (US)**

(72) Inventor: **Brown, Karen K.**
**10000 N.E. Harrison**
**Kansas City, MO 64155 (US)**
Inventor: **Bryant, Sharon A.**
**6727 Woodland**
**Shawnee, KS 66218 (US)**
Inventor: **Lewis, Kenneth S.**
**16637 W. 145th Street**
**Olathe, KS 66062 (US)**

(74) Representative: **Dänner, Klaus, Dr. et al**
**c/o Bayer AG Konzernverwaltung RP**
**Patentabteilung**
**D-5090 Leverkusen 1 Bayerwerk (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

BACKGROUND OF THE INVENTION

*Field:* This disclosure is concerned with the preparation of an immunogenic protein material from *Streptococcus equi* bacteria suitable as a vaccine against *Strep. equi* infection in equines using an enzymatic digestion and detergent treatment.

*Prior Art: Streptococcus equi* is classified as a Lancefield Group C Streptococcus. See, for example, Bergey's Manual of Determinitive Bacteriology (8th Ed.), p. 498 (1974). It is recognized as the causative agent of a severe respiratory disease of horses referred to as "Strangles". The disease is endemic in most parts of the world and epidemic in the United States. Race and show horses are particularly susceptible to repeated infections due to the stress of travel and exposure to new contacts. The disease begins with a mucopurulent nasal discharge, temperatures of 103°—106°F (39.4—41.1°C) and severe inflammation of the upper respiratory mucosa. It finally progresses to lymphadenitis and abscess formation which is sometimes severe enough to restrict air intake and cause suffocation of the animal. Strangles results in extensive loss of condition (loss of weight) as it often runs a course of 4—6 weeks.

Because of the debilitating and in some cases lethal effects of *Strep. equi* infections in horses, attempts have been made over the years to prepare *Strep. equi* vaccines which could be used for active immunization purposes. Unfortunately, *Strep. equi* preparations have been noted for their affinity for dermal tissue, producing severe swelling and even abscess formation at the injection site. These known reactivities have tended to discourage the development and/or commercial use of immunizing *Strep. equi* products. Two commercially available Strangles vaccines do exist, however. The first commercial product was a whole culture, chemically inactivated *Strep. equi* preparation (supplied by Ft. Dodge Corporation). The second commercial product was a cell free M-protein vaccine (available from Burroughs Wellcome Co.) described as "a concentrated, aluminum hydroxide-absorbed suspension of purified antigens derived from *Strep. equi*". The method by which this vaccine is prepared is thought to be described in U.S. Patent No. 3,793,150 and U.S. Patent No. 3,852,420. The purification of such "M-like proteins" from *Strep. equi* is also described in an article by J. B. Woolcock, Infect. and Immun., July 1974, p. 116—122. As used herein, the expression "M-like protein" means the immunogenic protein(s) of the *Strep. equi* organism which appears similar in molecular weight and activity to the M-protein of group A streptococci.

The theory that a protein on the cell wall of the *Strep. equi* organism, referred to as an M-like protein, is the antigenic portion of the bacteria has been discussed in articles by S. K. Srivastava and D. A. Barnum in the Can. J. Comp. Med., Vol. 46, p. 51—56, 1982 and in the Am. J. Vet. Res., Vol. 44, p. 41—45, 1983 and in articles by E. D. Erickson and N. L. Norcross in the Can. J. Comp. Med., Vol. 39, p. 110—115,1975. In all previous work, this M-like protein was extracted from the *Strep. equi* organism by subjecting the organism to low pH conditions (pH2) and high temperatures (95°—100°C) for a given time (10—15 minutes). This has been referred to as a "heat extraction" method of preparing the M-like protein. The protein precipitates under these conditions and is solubilized by raising the pH of the solution to pH 7 or above.

It was known to make equine strangles vaccine by extracting antigenic material from Streptococcus equi cultures. Phage associated lysin is mentioned to help in the preparation of antigens, but no details are given (US—P 3 852 420).

It was known from J. Exp. Med 144 (1976), p. 32—53 to extract M-proteins from group A streptococci by use of a non-ionic detergent.

It was known from Japanese published Specification Sho 57—209 228 that a hemolytic Streptococcus M-protein could be obtained by treating the bacteria culture with a cell wall dissolution enzyme which had no enzymic activity with respect to protein such as endo-N-acetylmuramidase (mutanolysin). The M-protein was then salted out from the supernatent.

We now have developed an improved method of removing the M-like protein from *Strep. equi* organisms, details of which are described herein.

We have now found that the antigenic M-like protein can be efficaciously removed from a *Strep. equi* culture in a two step process using mutanolysin digestion followed by treatment with an anionic detergent and that this extract can be used to prepare a vaccine effective in immunizing horses against infection by *Strep. equi.*

SUMMARY OF THE INVENTION

The procedure for our enzymatic extraction of Streptococcal M-like protein involves growth of a Streptococcal culture under growth-inducing conditions (e.g. at 37°C in a suitable media) followed by concentration of the cells (e.g. by centrifugation or filtration). The cell concentrate is either diluted or washed in a suitable buffer. Mutanolysin is then added to the cell concentrate and incubated at sufficient temperature and time for enzymatic lysis of part of the cell wall. Partial lysis means lysis sufficient to make the M-protein available for subsequent detergent extraction, but without deleterious effect on the M-protein. In general, we found this can be accomplished by exposing the *Strep. equi* culture to the lytic enzyme at 37°C for no more than about 24 hours at an enzyme concentration of about 1—10 units per ml of original culture volume. An anionic detergent such as sodium lauryl sulfate or dioctyl sodium sulfosuccinate is then added to the cell concentrate and allowed to incubate to complete the *Strep. equi* cell extraction treatment. Cells and cell debris are then removed by centrifugation or filtration and the final cell-

2

free antigen solution sterilized by filtration or chemical treatment. The cell-free antigen solution is immunogenic and useful in immunizing horses against infection by *Strep. equi* organisms and has the following characteristics: a molecular weight ranging from 25,000 to 75,000 daltons; heat stability to about 95°C; and trypsin sensitivity.

SPECIFIC EMBODIMENTS

The preferred bacteriolytic enzyme used in our method is mutanolysin (N-acetylmuramidase) which is obtained from the culture filtrate of *Streptomyces globisporus* and which is commercially available from Sigma Chemical Co., St. Louis, Mo. 63178 and Dainippon Pharmaceutical Co., Ltd., Osaka, Japan. Studies using mutanolysin as a method of lysing Streptococcal cell walls have been conducted for purposes other than M-like protein retrieval. Articles of these studies have been written by K. Yohagawa, et al in Antimicrobial Agents and Chemotherapy, August 1974, p. 156—165, G. B. Calandar and R. M. Cole in Infect. and Immun., June 1980, p. 1033—1037, and B. J. DeCueninck, et al, in Infect. and Immun., Feb. 1982, p. 572—582. Mutanolysin and other bacteriolytic enzymes (glycosidases) such as egg white lysozyme are thought to act on linear sequences of N-acetylglucosamines and N-acetylmuramic acid residues of the bacterial cell walls.

Example

1. *Strep. equi* deposited with American Type Culture Collection, Rockville, Md. 20852 as A.T.C.C. No. 39,506 was used. Grow *Strep. equi* in chemically defined medium (I. Van de Rijn, Infect. and Immun., 27: 444—448, 1980) at 37°C for 16 hours.

2. Concentrate *Strep. equi* cells to 10—50 fold using cross-flow filtration. Wash cells by addition of 0.1 M Trizma — HCl buffer with pH adjusted to 6.5 with NaOH.

3. Concentrate washed *Strep. equi* cells to 20—100 fold using cross flow filtration.

4. Add mutanolysin as a 5,000 unit/ml solution to concentrated cells achieving a final enzyme concentration of 5 units per ml original culture volume. Incubate at 37°C for 16 hours.

5. Ad 10% sodium lauryl sulfate to achieve a final concentration of .05%. Incubate at 37°C for 30 minutes.

6. Remove *Strep. equi* cells and cell debris by cross flow filtration or centrifugation.

7. Sterile filter effluent through .2 micron filter and hold at 4°C.

Antigen prepared according to the example was tested for potency via the previously-mentioned mouse combining power assay. In this assay, the antigenicity of the vaccine is measured by the increase in LD50 over the LD50 of the Antiserum Control. The greater this increase in LD50, the greater the antigenicity of the vaccine. Table 1 shows the results of vaccines tested via this assay. It should be noted that antigen diluted as much as 1:200 still demonstrates combining power.

## TABLE 1

### MOUSE COMBINING POWER RESULTS OF VACCINE PREPARATION

| Antigen Dilution Used To Prepare Vaccine | Mouse LD50 (Log) | LD50 Increase over Antiserum Control (Log) |
|---|---|---|
| 1:100 | 8.0 | 4.6 |
| 1:150 | 6.5 | 3.1 |
| 1:200 | 4.7 | 1.3 |
| Antiserum Control | 3.4 | - |
| Negative Control | 8.2 | - |

To prove that the enzyme-detergent treatment was the key factor contributing to antigen retrieval, the following experiment was conducted:

1. *Strep. equi* culture was grown and the cells were then harvested by centrifugation.

2. The cells were resuspended in buffer and aliquots of the suspension treated in the following manner: (In all cases, cells were removed following treatment by centrifugation and supernates filtered thru a 0.2 micron sterile filter.)

A. Suspension incubated at 50°C for 16 hours, then 0.05% sodium lauryl sulfate (SLS) added and suspension held at 37°C for 30 minutes.

B. Suspension incubated at 37°C for 30 minutes after addition of 0.05% SLS.

C. Suspension incubated at 37°C for 16 hours after addition of 0.05% SLS.

D. Suspension incubated at 50°C for 16 hours after addition of 5 units/ml mutanolysin.

E. Suspension incubated at 50°C for 16 hours after addition of 5 units/ml mutanolysin, followed by incubation at 37°C for 30 minutes after addition of 0.05% SLS.

F. Suspension heated at 95°C for 15 minutes after pH adjusted to 2.0. Suspension cooled and pH adjusted to 7.0.

Each of these preparations (A—F) were then tested in the mouse combining power assay to determine the amount of antigen released. The results (Table 2) show that detergent alone or in combination with heat (A, B, C) does not remove antigen. Mutanolysin incubated at 50°C (D) removes minimal quantity of antigen compared with the combination of Mutanolysin and detergent (E). In fact, this latter technique (E) appears to remove antigen as well as the hot acid extraction (heat extraction) method (F) described in U.S. Patent No. 3,793,150 and U.S. Patent No. 3,852,420 based on combining power results shown in this table.

## TABLE 2

### MOUSE COMBINING POWER RESULTS OF ANTIGEN PREPARATION

| Treatment of Cells | | Diln. of Preparation | Mouse LD50 (Log) | LD50 Increase over Antiserum Control (Log) |
|---|---|---|---|---|
| A. | 16 hours at 50° C + .05% SLS - 30 min. @ 37° C | 1:5 | <5 | <1.4 |
| B. | .05% SLS - 30 min @ 37° C | 1:5 | <5 | <1.4 |
| C. | .05% SLS - 16 hrs. at 37° C | 1:5 | <5 | <1.4 |
| D. | 5 units/ml mutanolysin 16 hrs. at 50° C | 1:5 | 5.0 | 1.4 |
| | | 1:10 | <5 | <1.4 |
| E. | 5 units/ml mutanolysin 16 hrs. at 50° C - .05% SLS - 30 min. at 37° C | 1:5 | 7.4 | 3.8 |
| | | 1:10 | 5.6 | 2.0 |
| F. | pH 2 - 95° C 15 minutes | 1:5 | 7.5 | 3.9 |
| | | 1:10 | 5.5 | 1.9 |
| | Antiserum Control | - | 3.6 | - |
| | Negative Control | - | 7.7 | - |

Further work with the mutanolysin-detergent method has recently shown that antigen removal is even more effective at 37°C than at 50°C. Therefore, the current process used to prepare the vaccines shown tested in Table 1 has been established at this preferred lower temperature of 37°C. To confirm the antigenicity of vaccines as measured in the mouse combining power assay, a vaccine efficacy study was conducted in equines using the vaccines shown in Table 1. Horses were given two doses of vaccine at 3 week intervals and then challenged with virulent *Strep. equi*. The efficacy of the vaccines was measured by the reduction in clinical symptoms observed post challenge. Clinical index values were assigned to Strangles symptoms for the purpose of efficacy evaluation. These clinical index values are shown in Table 3 and were assigned to each horse for each day symptoms were observed post challenge. Observations of the horses were made every other day for 49 days post challenge.

## TABLE 3

### CLINICAL INDEX VALUES ASSIGNED TO STRANGLES SYMPTOMS

| Symptom | Clinical Index Value |
|---|---|
| Abscess Formation | 20 points |
| White Blood Cell Count | |
| >50% increase | 5 points |
| >100% increase | 10 points |
| Temperature | |
| 102.0° F - 102.4° F | 1 point |
| 102.5° F - 103.9° F | 5 points |
| >104° F | 10 points |
| Nasal Discharge | |
| Moderate | 5 points |
| Heavy | 10 points |

The results of the vaccine efficacy study are shown in Table 4 as the accumulation of clinical index values assessed during the 49 day observation period. The reduction of clinical indices is also shown in Table 4 as the percent reduction of the indices of the vaccine groups as compared to the nonvaccinated control group. These data suggest that efficacious vaccines can be produced using enzyme-detergent extraction of *Strep. equi.*

5

### TABLE 4

*Strep. Equi* Efficacy Study
Clinical Indices

| Clinical Symptom | Vaccine 1:100 | Vaccine 1:150 | Vaccine 1:200 | NonVaccinated Controls |
|---|---|---|---|---|
| **Total** | | | | |
| Group Total | 819 | 1235 | 1586 | 2453 |
| Mean Value | 28.2 | 42.6 | 56.6 | 81.8 |
| % Reduction | 64% | 46% | 28% | - |
| **Abscesses** | | | | |
| Group Total | 180 | 420 | 680 | 1240 - |
| Mean Value | 6.2 | 14.5 | 24.3 | 41.3 |
| % Reduction | 85% | 65% | 41% | - |
| **WBC** | | | | |
| Group Total | 375 | 470 | 585 | 820 |
| Mean Value | 12.9 | 16.2 | 20.9 | 27.3 |
| % Reduction | 53% | 41% | 23% | - |
| **Temperature** | | | | |
| Group Total | 179 | 230 | 211 | 222 |
| Mean Value | 6.2 | 7.9 | 7.5 | 7.4 |
| % Reduction | 16% | 0% | 0% | - |
| **Nasal Discharge** | | | | |
| Group Total | 85 | 115 | 110 | 70 |
| Mean Value | 2.9 | 4.0 | 3.9 | 2.3 |
| % Reduction | 0% | 0% | 0% | - |

It is intended that the above specific Example should be construed as illustrative of the best mode to date of the invention disclosed and that the inventions disclosed should be limited only by the following claims.

**Claims**

1. A method of preparing a cell-free antigenic solution useful in immunizing horses against *Streptococcus equi* bacteria, the method comprising the steps of:
    a) growing *Streptococcus equi* bacteria under growth inducing conditions;
    b) exposing the bacteria of step a) to mutanolysin;
    c) extracting from the product of step b) with an anionic detergent immunogenic M-like protein(s) into the supernate;
    d) separating the soluble extracted M-like protein supernate from bacterial cells and cell debris;
    e) sterilizing the product of step d).

2. The method of claim 1 wherein in step b) the exposure is at 37°C for not more than about 24 hours at an enzyme concentration of 1—10 units per ml of original culture volume.

3. The method of claim 1 wherein the detergent of step c) is sodium lauryl sulfate and the exposure is at 37°C for not more than about 60 minutes at a detergent concentration of 0.01—0.10%.

## Patentansprüche

1. Verfahren zur Herstellung einer zellfreien antigenen Lösung, die geeignet ist zur Immunisierung von Pferden gegen Streptococcus equi-Bakterian, gekennzeichnet durch die folgenden Verfahrensschritte:

a) Züchten von Streptococcus equi-Bakterien unter das Wachstum induzierenden Bedingungen;

b) Einwirkung von Mutanolysin auf die Bakterien von Schritt a);

c) Extrahieren von immunogenem M-artigen Protein bzw. Proteinen aus dem Produkt von Schritt b) mit einem anionischen Detergens in den Überstand;

d) Abtrennen des Überstandes mit löslichem, extrahierten M-artigen Protein von Bakterienzellen und Zellresten;

e) Sterilisieren des Produktes von Schritt d).

2. Verfahren nach Anspruch 1, wobei im Schritt b) die Einwirkung bei 37°C für eine Zeitdauer von nicht mehr als 24 h bei einer Enzymkonzentration von 1 bis 10 Einheiten pro ml des ursprünglichen Kulturvolumens erfolgt.

3. Verfahren nach Anspruch 1, wobei das Detergens in Schritt c) Natriumlaurylsulfat darstellt und die Einwirkung ca. 60 Minuten bei 37°C und einer Detergens-Konzentration von 0,01 bis 0,10% erfolgt.

## Revendications

1. Un procédé de préparation d'un solution antigénique exempte de cellules utilise pour immuniser les cheavaux contre les bactéries *Streptococcus equi*, le procédé comprenant les étapes suivantes:

a) on fait pousser les bactéries *Streptococcus equi* dans des conditions provoquant la croissance;

b) on expose les bactéries de l'étape a) à la mutanolysine;

c) on extrait la ou les protéines du type M immunogènes dans le surnageant à partir du produit de l'étape b) par un détergent anionique;

d) on sépare le surnageant de protéine du type M extraite soluble des cellules bactériennes et débris cellulaires;

e) on stérilise le produit de l'étape d).

2. Le procédé selon la revendication 1, dans lequel, dans l'étape b), l'exposition est à 37°C pendant pas plus d'environ 24 heures à une concentration d'enzyme de 1—10 unités par ml du volume de la culture initiale.

3. Le procédé selon la revendication 1, dans lequel le détergent de l'étape c) est le laurylsulfate de sodium et l'exposition est à 37°C pendant pas plus d'environ 60 minutes à une concentration de détergent de 0,01—0,10%.